# EUROPEAN PATENT APPLICATION

(11) **EP 3 403 649 A1**
(43) Date of publication of application: **21.11.2018**
(21) Application number: 17171351.4
(22) Date of filing: 16.05.2017
(51) Int. Cl.: A61K 31/00, A61K 31/20, A61P 29/00

(54) **INHIBITORS AND ANTAGONISTS OF GPR84 FOR THE TREATMENT OF ENDOMETRIOSIS**

(71) Applicant: Bayer Pharma Aktiengesellschaft, 13353 Berlin (DE)
(72) Inventor: Sacher, Frank, 10409 Berlin (DE); Obendorf, Maik, 99425 Weimar (DE); Langer, Gernot, 14612 Falkensee (DE); Martinez Estrada, Fernando, Godalming, GU8 5JD (GB); Oppermann, Udo, Oxford, OX1 5DQ (GB); Shang, Catherine, Melbourne, 3142 (AU)
(74) Representative: BIP Patents

(57) **Abstract**

The present invention relates to an antagonist or inhibitor of G protein-coupled receptor 84 (GPR84) for use in the treatment and/or prevention of a patient suffering from, at risk of developing, and/or being diagnosed for endometriosis, as well as methods of screening for such antagonist or inhibitor as well as methods for diagnosis.

## Description

The present invention relates to the use of inhibitors and antagonists of human GPR84, for the treatment and/or prevention of endometriosis. The inhibitors and antagonists according to the invention are antibodies, nucleic acids, aptamers, or small molecules. The invention also provides assays and screening technologies to find such inhibitors and antagonists.

### Background of the Invention

Endometriosis affects 5-10% of women of reproductive age and is defined as the growth of endometrial tissue in ectopic locations, found primarily within the pelvic cavity. This extra-uterine growth is accompanied by infiltration of pro-inflammatory macrophages as cardinal sign of disease leading to a chronically inflamed microenvironment and contributing to chronic pain symptoms.

Peritoneal lesions and fluids of women with endometriosis are characterized by infiltration of leukocytes including Natural Killer (NK) cells and phagocytic macrophages [1, 2] besides T-lymphocytes, which together contribute to a pro-inflammatory milieu that characterizes the disease. Besides the presence of infiltrating leukocytes, endometriosis is characterized by elevated levels of inflammatory mediators such as cytokines and growth factors including TNFalpha and IL-1beta [3], two pro-algesic mediators.

Medium-chain fatty acids (MCFFA) are fatty acids with tails of 6 to 12 carbons and can activate GPR84 [4]. There are two sources of FAs for animal metabolism, exogenously-derived (dietary) FAs and endogenously-synthesized FAs. The biosynthesis of the latter is catalyzed by FASN [5]. MCFFAs stimulate release of IL6 in chondrocytes [6] and myristic acid increases IL6 and IL8 levels in human coronary arterial smooth muscle (HCASM) and endothelial (HCEC) cells [7].

GPR84 belongs to the group of Free Fatty Acid (FFA) receptors [8]. The group of FFA receptors consist of 4 GPCRs (FFA1-FFA2) and the new members GPR42 and GPR84. FFA receptor are involved in biological processes such as metabolic and immune functions [8].

In contrast to all other FFA receptors which have a broader expression pattern, GPR84 has been described to be expressed primarily in various leukocyte populations [8].

Until now there is no cure for endometriosis, yet there are two types of interventions: treatment of pain and treatment of endometriosis-associated infertility. Regarding pharmaceutical therapies, hormonal therapies (progesterone, progestins, xenoestrogens, oral contraceptives, Danazol (Danocrine), gestrinone, Gonadotropin-releasing hormone (GnRH) agonists or aromatase inhibitors), NSAIDs, opioids, or Pentoxifylline, can be administered, while further surgical removal of the endometrium, adhesions, resection of endometriomas, and restoration of normal pelvic anatomy can be used. Pharmaceutical and surgical interventions produce roughly equivalent pain-relief benefits. Recurrence of pain was found to be 44 and 53 percent with medicinal and surgical interventions, respectively (24).

However, treatment options are both limited and unsatisfactory today, leaving a demand for providing better treatment options in terms of efficacy, sustainability, reduced side effects, patient compliance and the like.

### Summary of the invention

These and further objects are met with methods and means according to the independent claims of the present invention. The dependent claims are related to specific embodiments.

### Embodiments of the invention

Before the invention is described in detail, it is to be understood that this invention is not limited to the particular component parts or structural features of the devices or compositions described or process steps of the methods described as such devices and methods may vary. It is also to be understood that the terminology used herein is for purposes of describing particular embodiments only, and is not intended to be limiting. The mere fact that certain measures are recited in mutually different dependent claims does not indicate that a combination of these measures cannot be used to advantage. Any reference signs in the claims should not be construed as limiting the scope. It must be noted that, as used in the specification and the appended claims, the singular forms "a," "an" and "the" include singular and/or plural referents unless the context clearly dictates otherwise. Further, in the claims, the word "comprising" does not exclude other elements or steps. The mere fact that certain measures are recited in mutually different dependent claims does not indicate that a combination of these measures cannot be used to advantage.

It is moreover to be understood that, in case parameter ranges are given which are delimited by numeric values, the ranges are deemed to include these limitation values.

It is further to be understood that embodiments disclosed herein are not meant to be understood as individual embodiments which would not relate to one another. Features discussed with one embodiment are meant to be disclosed also in connection with other embodiments shown herein. If, in one case, a specific feature is not disclosed with one embodiment, but with another, the skilled person would understand that does not necessarily mean that said feature is not meant to be disclosed with said other embodiment. The skilled person would understand that it is the gist of this application to disclose said feature also for the other embodiment, but that just for purposes of clarity and to keep the specification in a manageable volume this has not been done.

"Inhibition" or "reduction" in context with the present invention is to be understood as reducing the measured outcome by at least 20% as compared to a control, e.g., a non-treated control, preferably by at least 50%, more preferably by at least 75%. Suited controls are evident for the skilled person when considering the teaching of the present disclosure. Suitable assays to determine said inhibition or reduction are readily available to the skilled person from the pertinent literature. In one embodiment, the cAMP release assays referred to herein below are being used to determine said inhibition or reduction.

"Inhibition" and "antagonizing" as well as "inhibitor" and "antagonist" are used interchangeably herein.

In a preferred embodiment the term "inhibition" refers to specific inhibition. "Specific inhibition" denotes an inhibition of the named receptor while other receptors are not significantly inhibited, preferably other G-protein coupled receptors (GPCR). In a preferred embodiment "specific inhibition of GPR84" refers to an inhibition of GPR84 activity while not significantly inhibiting one or more membrane transporters or other G-protein coupled receptors (GPCR), preferably one or more selected from the group consisting of Norepinephrine (NET), Dopamine (DAT), Potassium Channel hERG, Potassium Channel (KATP), GPR40. In a preferred embodiment "other G-protein coupled receptors (GPCR)" refers to GPR40.

In a preferred embodiment specific inhibition of GPR84 refers to a inhibition of GPR84 activity by at least 20% as compared to a control, preferably by at least 50%, more preferably by at least 75%, while inhibiting the activity of one or more membrane transporters selected from the group consisting of other G-protein coupled receptors (GPCR); preferably selected from the group consisting of Norepinephrine (NET), Dopamine (DAT), Potassium Channel hERG, Potassium Channel (KATP), and GPR40; by at most 75%, more preferably by at most 50%, yet more preferably by at most 10%. Suitable assays to determine said inhibition or reduction are readily available to the skilled person from the pertinent literature. In one embodiment, the cAMP release assays referred to herein below are being used to determine said inhibition or reduction. Inhibition of the other receptors can be assessed as exemplified in the Example's experiments.

According to one embodiment of the invention, an antagonist or inhibitor of G protein-coupled receptor 84 (GPR84) is provided for use in the treatment and/or prevention of a patient
- suffering from,
- at risk of developing, and/or
- being diagnosed for
endometriosis.

The inventors of the present invention found, surprisingly, that GPR84 is expressed in inflammatory cell types and in diseased tissue of endometriosis patients, and also showed that GPR84 inhibition has an alleviating effect on this condition, such as inflammatory pain. Especially, GPR84 inhibition reduced pro-inflammatory and pro-algesic mediators, such as IL-1beta and TNFalpha, within an *in vitro* endometriosis model. Hence, treatment of endometriosis and its associated symptoms by using a method to reduce endometriosis associated elevated levels of both TNFalpha and IL-1beta levels has the potential to treat symptoms and disease progression of endometriosis. In a particular embodiment the antagonist or inhibitor is for treating endometrioses-associated inflammatory pain.

Furthermore, it is shown herein that GPR84 agonists, e.g., medium chain fatty acids (MCFA), are present in endometriotic tissue and lesions, and contribute to the elevation inflammatory mediators which characterize the disease

As used herein, the term "antagonist or inhibitor of GPR84" refers to a compound that is capable of reducing or inhibiting either directly or indirectly the activity of GPR84, preferably by at least 20%, preferably by at least 50%, more preferably by at least 75%. Suitable assays to determine said inhibition or reduction are readily available to the skilled person from the pertinent literature. In one embodiment, the cAMP release assays referred to herein below are being used to determine said inhibition or reduction.

Preferably, the antagonist or inhibitor of GPR84 is a selective inhibitor for GPR84 as defined above.

Such GPR84 activity is for example a downstream effector function of GPR84, like, e.g., inflammatory mediator release in human and murine PBMC or macrophages.

Said reduction or inhibition may be accomplished, for example, by reducing or inhibiting endogenous and synthetic GPR84 agonist binding. Said reduction or inhibition of agonist binding may occur, e.g., by competitive or allosteric binding to the GPR84 protein.

Preferred agonists of GPR84 are medium chain free fatty acids (MCFFA), preferably with carbon chain lengths of 9 to 14 (see 25, 19, which are incorporated herein by reference). These include, *inter alia,* 2-OH decanoic acid and decanoic acid, which are particularly preferred GPR84 agonists.

An artificial agonist for GPR84 is 6-n-octylaminouracil (6-OAU) (see 17, incorporated herein by reference). Other preferred artificial agonists are described; e.g. 3,3'-diindolylmethane (see 19, incorporated herein by reference), and Embelin (see WO2007027661 A1, incorporated herein by reference). The following table shows some selected agonist according to the invention:

| **ligand/agonist** | **structure** |
|---|---|
| Decanoic acid | 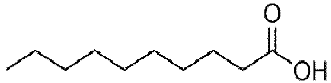 |
| 2-Hydroxydecanoic acid | 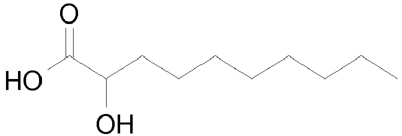 |
| 6-n-octylaminouracil | 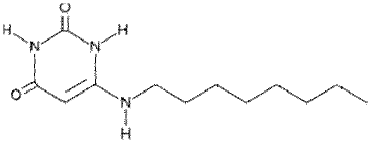 |
| 3,3'-diindolylmethane | 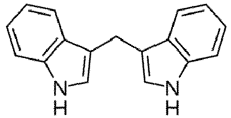 |
| Embelin | 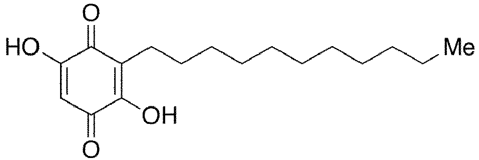 |

In one embodiment, the antagonist is an antibody, preferably a monoclonal antibody, specifically binding to GPR84, or is an antigen-binding fragment or derivative thereof. The antibody is preferably a monoclonal antibody (mAb).

As used herein, the term "monoclonal antibody", shall refer to an antibody composition having a homogenous antibody population, i.e., a homogeneous population consisting of a whole immunoglobulin, or an antigen binding fragment or derivative thereof. Particularly preferred, such antibody is selected from the group consisting of IgG, IgD, IgE, IgA and/or IgM, or a fragment or derivative thereof.

As used herein, the term "fragment" shall refer to fragments of such antibody retaining target binding capacities, e.g.
- a CDR (complementarity determining region),
- a hypervariable region,
- a variable domain (Fv),
- an IgG heavy chain (consisting of VH, CH1, hinge, CH2 and CH3 regions),
- an IgG light chain (consisting of VL and CL regions), and/or
- a Fab and/or F(ab)₂.

As used herein, the term "derivative" shall refer to protein constructs being structurally different from, but still having some structural relationship to the common antibody concept, e.g., scFv, Fab and/or F(ab)₂, as well as bi-, tri- or higher specific antibody constructs or monovalent antibodies, and further retaining target binding capacities. All these items are explained below.

Other antibody derivatives known to the skilled person are Diabodies, Camelid Antibodies, Nanobodies, Domain Antibodies, bivalent homodimers with two chains consisting of scFvs, IgAs (two IgG structures joined by a J chain and a secretory component), shark antibodies, antibodies consisting of new world primate framework plus non-new world primate CDR, dimerised constructs comprising CH3+VL+VH, and antibody conjugates (e.g. antibody or fragments or derivatives linked to a toxin, a cytokine, a radioisotope or a label). These types are well described in literature and can be used by the skilled person on the basis of the present disclosure, with adding further inventive activity.

As discussed above, GPR84 is sufficiently specified to enable a skilled person to make an antibody, such as a monoclonal antibody thereagainst. Routine methods encompass hybridoma, chimerization/humanization, phage display/transgenic mammals, and other antibody engineering technologies.

Methods for the production of a hybridoma cell have been previously described (see [30], incorporated herein by reference). Essentially, e.g., a mouse is immunized with a human GPR84 protein, followed by B-cell isolation from said mouse and fusion of the isolated B-cell with a myeloma cell.

Methods for the production and/or selection of chimeric or humanized mAbs are known in the art. Essentially, e.g., the protein sequences from the murine anti-GPR84 antibody which are not involved in target binding are replaced by corresponding human sequences. For example, US6331415 by Genentech describes the production of chimeric antibodies, while US6548640 by Medical Research Council describes CDR grafting techniques and US5859205 by Celltech describes the production of humanised antibodies. All of these disclosures are incorporated herein by reference.

Methods for the production and/or selection of fully human mAbs are known in the art. These can involve the use of a transgenic animal which is immunized with human GPR84, or the use of a suitable display technique, like yeast display, phage display, B-cell display or ribosome display, where antibodies from a library are screened against human GPR84 in a stationary phase.

In vitro antibody libraries are, among others, disclosed in US6300064 by MorphoSys and US6248516 by MRC/Scripps/Stratagene. Phage Display techniques are for example disclosed in US5223409 by Dyax. Transgenic mammal platforms are for example described in EP1480515A2 by TaconicArtemis. All of these disclosures are incorporated herein by reference.

IgG, scFv, Fab and/or F(ab)₂ are antibody formats well known to the skilled person. Related enabling techniques are available from the respective textbooks.

As used herein, the term "Fab" relates to an IgG fragment comprising the antigen binding region, said fragment being composed of one constant and one variable domain from each heavy and light chain of the antibody.

As used herein, the term "F(ab)₂" relates to an IgG fragment consisting of two Fab fragments connected to one another by one or more disulfide bonds.

As used herein, the term "scFv" relates to a single-chain variable fragment being a fusion of the variable regions of the heavy and light chains of immunoglobulins, linked together with a short linker, usually serine (S) or glycine (G). This chimeric molecule retains the specificity of the original immunoglobulin, despite removal of the constant regions and the introduction of a linker peptide.

Modified antibody formats are for example bi- or trispecific antibody constructs, antibody-based fusion proteins, immunoconjugates and the like. These types are well described in literature and can be used by the skilled person on the basis of the present disclosure, with adding further inventive activity. Furthermore, also monovalent antibodies have been previously described in US 2004/0033561 A1 (referred to therein as monobodies) or WO2007048037; both of which are incorporated herein by reference.

Finding a suitable antibody, or fragment or derivative, that is capable of acting as an antagonist of GPR84, e.g., by binding to its active center, is hence a matter of routine for the skilled person, based on the public availability of the amino acid sequences of GPR84.

Polyclonal antibodies against GPR84 for scientific research are commercially available, e.g., from ThermoFischer (PA5-32843), Santa Cruz Biotechnology (H-300, D-15) or Novus Biologicals (NBP1-84768), to name a few, hence demonstrating that the skilled person is capable of making a therapeutic antibody against GPR84.

Because GPR84 is a 7-transmembrane receptor protein, the antibody or its fragment or derivative may bind to an extracellular or an intracellular domain of GPR84. In the latter case the antibody or its fragment or derivative needs to be funneled or trafficked into the intracellular space. Routine technologies are available for this purpose, which are disclosed elsewhere (26 - 28, incorporated herein by reference).

In one embodiment, the antagonist or inhibitor is a nucleic acid inhibitor. This embodiment can for example comprise a nucleic acid molecule that specifically binds to at least a substretch of a polynucleotide which encodes a GPR84 protein.

Preferably, said polynucleotide encodes for a GPR84 protein which comprises an AA sequence as disclosed in Uniprot under reference Number Q9NQS5, preferably according to SEQ ID NO: 1.

Many of the nucleic acid inhibitor approaches rely on base pairing of such inhibitory nucleic acid molecule with the target sequence, i.e., the polynucleotide encoding for a GPR84. Said polynucleotide encoding for a GPR84 can either be mRNA or genomic DNA.

Preferably, said inhibitor is selected from the group consisting of a siRNA, a shRNA, crRNA or a guide RNA.

siRNA are short artificial RNA molecules which can be chemically modified to enhance stability. Because siRNA are double-stranded, the principle of the 'sense' and the 'antisense' strand also applies. The sense strands have a base sequence identical to that of the transcribed mRNA and the antisense strand has the complementary sequence. Technically, a siRNA molecule administered to a patient is bound by an intracellular enzyme called Argonaut to form a so-called RNA-induced silencing complex (RISC). The antisense strand of the siRNA guides RISC to the target mRNA, where the antisense strand hybridizes with the target mRNA, which is then cleaved by RISC. In such way, translation of the respective mRNA is interrupted. The RISC can then cleave further mRNAs. Delivery technologies are e.g. disclosed elsewhere ([29], incorporated herein by reference) Finding a suitable sequence for the siRNA is a matter of routine for the skilled person, based on the public availability of the different mRNA isoforms of GPR84.

shRNA is an artificial RNA molecule with a tight hairpin turn that can be used to silence target gene expression via RNA interference (RNAi). shRNA can be delivered to cells, e.g., by means of a plasmid or through viral or bacterial vectors. shRNA is an advantageous mediator of RNAi in that it has a relatively low rate of degradation and turnover. The respective plasmids comprise a suitable promoter to express the shRNA, like a polymerase III promoter such as U6 and H1 or a polymerase II promoter. Once the plasmid or vector has integrated into the host genome, the shRNA is transcribed in the nucleus. The product mimics pri-microRNA (pri-miRNA) and is processed by Drosha. The resulting pre-shRNA is exported from the nucleus by Exportin 5. This product is then processed by Dicer and loaded into the RNA-induced silencing complex (RISC), after which the same silencing follows as in siRNA. Finding a suitable sequence for the shRNA is a matter of routine for the skilled person, based on the public availability of the different mRNA isoforms of GPR84.

The first nucleic acid molecule can be the guide RNA of a CRISPR Cas system (31), which guide RNA comprises a target-specific crRNA ("small interfering CRISPR RNA") capable of hybridizing with a genomic strand of the GPR84 gene (or, the first nucleic acid molecule can be the crRNA alone). The guide RNA/crRNA is capable of directing the Cas enzyme, which is an endonuclease, to the GPR84 gene, where the Cas enzyme carries out sequence specific strand breaks. By creating one or more double strand breaks, the GPR84 gene hence can be silenced. To use said system for in vivo gene silencing of GPR84, a dedicated delivery technology is required, which comprise a delivery vehicle such as lipid nanoparticles, as for example discussed elsewhere (32) Finding a suitable sequence for the crRNA comprised in the guide RNA is a matter of routine for the skilled person, based on the public availability of the genomic sequence of the GPR84 gene.

In another embodiment, said first nucleic acid molecule can also the guide RNA of a CRISPR Cpf1 system (33), which guide RNA comprises a target-specific crRNA ("small interfering CRISPR RNA"). Similar to CRISPR/Cas, the guide RNA is capable of directing the Cpf1 enzyme, which is an endonuclease, to the GPR84 gene. As regards technical considerations, e.g., delivery for *in vivo* applications and finding of the suitable sequence for the first nucleic acid molecule, similar aspects as with CRISPR/Cas apply.

Further embodiments of the CRISPR technology are currently under development, with different endonucleases. However, all these approaches use a target-specific RNA (the guide RNA or crRNA as in CRISPR Cas) that hybridizes with a target sequence. In all these cases, the target-specific RNA qualifies as the first nucleic acid molecule in the meaning of the preferred embodiment discussed herein. As regards technical considerations, e.g., delivery for *in vivo* applications and finding of the suitable sequence for the first nucleic acid molecule, similar aspects as with CRISPR Cas apply.

microRNA (abbreviated miRNA) is a small non-coding RNA molecule (containing about 22 nucleotides) found in plants, animals and some viruses, that functions in RNA silencing and post-transcriptional regulation of gene expression.

In one embodiment, the antagonist or inhibitor is an aptamer. Aptamers are oligonucleotides that have specific binding properties for a pre-determined target. They are obtained from a randomly synthesized library containing up to 10¹⁵ different sequences through a combinatorial process named SELEX ("Systematic Evolution of Ligands by EXponential enrichment"). Aptamer properties are dictated by their 3D shape, resulting from intramolecular folding, driven by their primary sequence. An aptamer3D structure is exquisitely adapted to the recognition of its cognate target through hydrogen bonding, electrostatic and stacking interactions. Aptamers generally display high affinity (K_{d} about micromolar (µM) for small molecules and picomolar (pM) for proteins).

An overview on the technical repertoire to generate target specific aptamers is given, e.g., in 34, which is incorporated herein by reference. Aptamers can also be delivered into the intracellular space, as disclosed in 35 (incorporated herein by reference).

Finding a suitable aptamer that is capable of acting as an antagonist of GPR84, e.g., by binding to its active center, is hence a matter of routine for the skilled person, based on the public availability of the amino acid sequences of the different GPR84 isoforms.

In another particularly preferred embodiment, the antagonist or inhibitor is a small molecule. Preferably, said small molecule is an organic molecule, and/or said small molecule has a molecular weight of smaller ≤ 550 DA, preferably ≤ 500 DA, more preferably ≤ 450 DA.

Preferably, the GPR84 protein which is inhibited or antagonized by the antibody, aptamer or small molecule comprises an AA sequence as disclosed in Uniprot under reference Number Q9NQS5, preferably according to SEQ ID NO: 1.

In a preferred embodiment of the invention the antagonist or inhibitor reduces or inhibits agonist-induced GPR84 activation by at least 20%, more prefereably by at least 50%, yet more preferred by at least 75%. Suitable assays to determine said inhibition or reduction are readily available to the skilled person from the pertinent literature. In one embodiment, the cAMP release assays referred to herein below are being used to determine said inhibition or reduction.

According to one embodiment of the invention, the antagonist or inhibitor inhibits agonist-induced GPR84 activation, preferably
- embelin-induced GPR84 activation with an IC₅₀ value of 5 mM or less, preferably 10 µM or less, more preferably 9.4 nM or less, and/or
- 2-OH decanoic-acid-induced GPR84-activation with an IC₅₀ value of 5 mM, preferably 10 µM or less or less, most preferably of 16 nM or less.

Preferably, embelin-induced GPR84 activation and/or 2-OH decanoic-acid-induced GPR84-activation is determined in a GPR84 cAMP release assay.

Inhibition of agonist-induced GPR84 activation by a potential inhibitor may be for example tested in a cAMP release assay according to the following principles:
GPR84 activity represses cAMP production in cells. Inhibition of GPR84, hence, results in increased cAMP content in cells. However, when testing the inhibitory potential of a substance by determining effects on cAMP levels, it has to be made sure that the effects observed are GPR84-specific rather than a general cAMP inducing effect. A preferred option is to investigate whether a substance (potential inhibitor) reverses the inhibitory effect of a GPR84 agonist. Agonists of Galphai signalling by GPR84 inhibit membrane adenylyl cyclases-induced cellular cAMP production. The addition of a GPR84 inhibitor (or GPR84 antagonist) results in an (re)-increase of membrane adenylyl cyclases-induced cAMP production in the presence of a GPR84 agonist. Hence, antagonistic effects on GPR84 result in increased cAMP content in the cell. The antagonistic effect of a potential inhibitor is preferably tested by its exposure to a in the presence of a GPR84 agonist and a membrane adenylyl cyclases-activating agent, such as forskolin. Any other compound or substance unspecifically increasing cAMP levels through membrane adenylyl cyclases-activation may be employed as well. One or more compounds preventing degeneration of cAMP may be added to ensure sufficient accumulation of this second messenger. Such compounds include phosphodiesterase inhibitors, e.g 3-Isobutyl-1-methylxanthine (IBMX).

The skilled person will acknowledge that GPR84-specific inhibition is determined, as the effect on agonistic GPR84 induction is assessed, *supra.* To further strengthen GPR84-specificity, cells overexpressing GPR84 may be used, e.g. but not limited to *"cAMP HunterTM CHO-K1 GPR84 Gi cells"* (DiscoverX; # 95-0158C2).

By determining cAMP content in cells contacted with the potential GPR84 inhibitor as outlined above and comparing it to the appropriate controls, it is possible to determine antagonistic effects. An increase in cAMP content as compared to the control indicates GPR84 antagonistic effects and qualifies the compound as an inhibitor. Preferably, a substance is qualified as an inhibitor of GPR84, if it increases the cAMP content of a cell contacted with the potential inhibitor, an agonist of GPR84, an activator of membrane adenylyl cyclases-induced cAMP production, and a phosphodiesterase inhibitor by at least 20% as compared to the appropriate control without the potential inhibitor.

The skilled person is able to detect cAMP content (levels) in a cell using common skills. Common techniques may for example employ a competitive assay. In principle, a detectable tracer is brought into contact with a cAMP binding agent in the presence of a sample the cAMP content of which shall be determined, wherein the detectable tracer binds to the cAMP binding agent competitively with cAMP. The competitive assay is to be constructed as to allow detection of the relative amount of tracer bound to the cAMP binding agent, e.g. by using compatible dyes for a fluorescence resonance energy transfer (FRET) assay. In the presence of the sample, the cAMP contained in the sample competes with tracer in binding to the cAMP binding agent. If the sample is a cell, the cell may be subject to lysis in order to release the intracellular cAMP content before applying it to the assay. The detected amount of tracer bound to the cAMP binding agent is inversely proportional to the cAMP content in the sample. In other words, intense signals for tracer binding to the cAMP binding agent indicate a low cAMP content in the sample, and - *vice versa -* weaker signals for tracer binding to the cAMP binding agent indicate a higher cAMP content in the sample. Maximum signal is obtained in the absence of cAMP. The decrease in cAMP content within a cell may hence be determined by comparing the cAMP content in a cell after addition of the test inhibitor as compared to a negative control and/or before addition of said potential inhibitor in a competitive assay. Detecting more cAMP binding to the cAMP binding agent in samples exposed to the potential inhibitor indicates inhibitory (antagonistic) effect of said potential inhibitor on GPR84. Weaker signals in the competitive assay for the samples treated with the potential inhibitor as compared to the controls in the competitive assay therefore indicate inhibitory effect of the the potential inhibitor on GPR84.

Quantification of the actual cAMP amounts may for example be achieved with the help of a competition assay based on a fluorescent dye-labelled cAMP tracer (e.g. cAMP-d2, (Cisbio; cAMP femto 2 Kit; # 62AM5PEJ ...]) and cAMP binding agents, such as labelled anti-cAMP antibodies, e.g. Eu-cryptate labelled anti-cAMP antibody (Cisbio; cAMP femto 2 Kit; # 62AM5PEJ). Upon cell lysis any cellular cAMP present competes for binding of the tracer to the antibody. Such signal may for example be measured using FRET, if compatible fluorescent dyes for the tracer and the cAMP binding agent are used. Depending on the dyes, different emissions and excitation wavelength are used as evident for the skilled person. For the combination of d2/Eu-cryptate, e.g., *supra,* FRET induced emissions at 665nm and 620nm are measured following excitation at 337 nm with an appropriate high-throughput fluorescence (HTRF) reader (e.g. RubiStar; Berthold Industries). Given the competitive nature of the detection system, agonist treatment results in lower cAMP levels and increases the HTRF signal. Any decrease in the HTRF signal in the presence of forskolin, agonist and antagonist is indicative of the abrogation of the inhibitory Galphai signalling by GPR84, and hence for the inhibitory effect on GPR84.

An exemplary procedure to detect inhibition of a substance may be conducted as follows:
1. Preparation of assay ready frozen cells
   For the resuscitation of frozen cells (cAMP HunterTM CHO-K1 GPR84 Gi cells; DiscoverX; # 95-0158C2) one vial is taken out of the liquid nitrogen storage and rapidly thawed in a water bath at 37°C for some 1 to 3 minutes. Immediately afterwards the cells are transferred carefully by decanting into a 50 ml Falcon Tube containing 15 ml of pre-warmed full cell culture medium (HAM's F12; 10 % FCS; no selection markers). Gentle swirling is used to assist their resuspension. In a next step, the cells are harvested straight away via low g centrifugation for 5 minutes (Hereaus Multifuge 4 KR; 400 rpm; rcf = 44g) to avoid over-compacting the cell pellet. Gentle decanting is used to get rid of the medium. Finally assay readiness is achieved by carefully re-suspending the cell pellet in 5 ml of the final assay medium (HAM's F12; 10 % FCS; no selection markers). In a last step, cell viability and the actual cell number is determined (Omni Life Science; Casy Cell Counter/Analyzer) prior to correcting the volume for the final cell number to be seeded, i.e. 2500 cells/µl. Cell seeding is achieved either with the help of multi-channel pipettes or appropriate bulk reagent dispensers.
2. Test for Agonism (384-well format):
   2 µl's of assay ready frozen cells are directly seeded into the wells of a non-sterile 384-well plate (Greiner; white; # 784075; 5000 cells/well). Immediately afterwards 2 µl's of a 2 X agonist/2 X EC80 Forskolin (final concentration = 8 µM, Sigma; #F6886) solution are added, which is prepared in assay medium (HAM's F12; 10 % FCS; 2 mM Glutamin; no selection markers). Following 60 minute of incubation time 2 µl' of the cAMP-d2 tracer as provided (Cisbio; cAMP femto 2 Kit; # 62AM5PEJ) are added followed by a separate addition of 2 µl's of the anti-cAMP antibody containing in cell lysis buffer (detection reagents prepared according to the manufacturers specifications). All additions are performed using either multi-channel pipettes (ThermoFisher/Thermo Scientific:E1-ClipTipTM; # 4671010) or bulk reagent dispensers (ThermoFisher Scientific: Multidrop Combi). Time resolved FRET (TR-FRET) signals are determined 60 minutes later using an appropriate HTRF reader (BMG Labtech: Pherastar).
3. Test for Antagonism (384-well format):
   In a first step, 1 µl of a 4 X antagonist solution in the predefined test range (prepared in assay medium; HAM's F12; 10 % FCS; 2 mM Glutamin; no selection markers) is added into the wells of a non-sterile 384-well plate (Greiner; white; # 784075; Immediately afterwards 2 µl's of assay ready frozen cells are directly seeded into the plate (5000 cells/well)). The cells are incubated in the presence of the potential inhibitor (antagonist) for 30 minutes prior to adding 1 µl of a 4 X agonist EC80 (final concentrations: Embelin = 6.00*10⁻⁶ M, 6-n-octylaminouracil = 1.34*10⁻⁷M) / 4 X EC80 Forskolin (final concentration = 8 µM) solution prepared in assay medium as well. Following 60 minute of incubation 2 µl of the cAMP-d2 tracer are added followed by the addition of 2 µl's of the anti-cAMP antibody containing in cell lysis buffer (detection reagents prepared according to the manufacturers specifications). All additions are performed using either multi-channel pipettes (ThermoFisher/Thermo Scientific:E1-ClipTipTM; # 4671010) or bulk reagent dispensers (ThermoFisher Scientific: Multidrop Combi). Time resolved FRET (TR-FRET) signals are determined 60 minutes later using an appropriate HTRF reader (BMG Labtech: Pherastar).

In one embodiment of the invention, the antagonist or inhibitor according to the invention is a member of one of the groups selected from the list comprising:
- Dihydropyridoisoquinolinone
- Dihydropyrimidinoisoquinolinone

Preferably, said antagonist or inhibitor is one of the Dihydropyridoisoquinolinones disclosed in any of US2016244442 A1, WO2016169911 A1, US2016039807 A1, and/or WO2015197550 A1 the contents of which are incorporated by reference herein. Likewise preferably, said antagonist or inhibitor is one of the Dihydropyrimidinoisoquinolinones disclosed in WO2014095798 A1, the contents of which are incorporated by reference herein. In a particularly preferred embodiment, said antagonist or inhibitor one of the following:

| | |
|---|---|
| 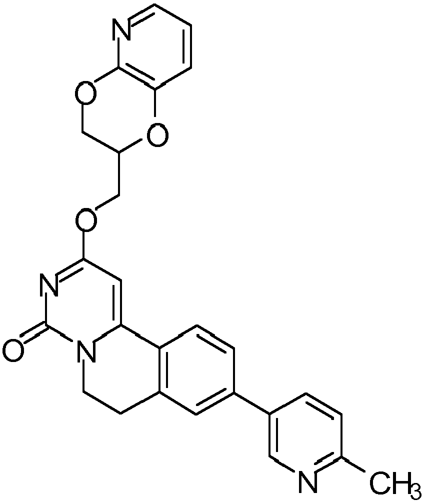 | Compound 139 of WO 2014095798 A1 (see para [0380] therein) |

In one embodiment of the present invention, the endometriosis is characterized by
- Increased presence of medium chain free fatty (MCFFA) acids in ectopic lesions, as compared to eutopic endometrium
- increased expression of GPR84 in monocytes and/or macrophages taken from an endometriosis patient, as compared to monocytes and/or macrophages taken from a control patient
- larger fraction of GPR84 positive cells in monocytes and/or macrophages taken from an endometriosis patient, as compared to monocytes and/or macrophages taken from a control patient.

Therein, the term MCFFA encompasses, preferably, C₉-C₁₄ fatty acids, preferably decanoic acid and/or myristic acid, while the term ACOT encompasses, preferably, ACOT4 and ACOT9.

The invention further provides a method of characterizing a gynaecologic condition as being endometriosis, said method providing the following steps:
a) taking a sample (smear, biopsy, scrape) from an ectopic endometrial lesion of a patient,
b) determining and/or quantifying the presence or absence of medium chain free fatty acids (MCFA), in that sample, and
c) comparing at least one of the obtained parameters with the respective (control) parameter (i) obtained from eutopic endometrium of a control patient or the same patient, or (ii) taken from suitable literature or databases,
wherein increased presence of MCFFA as compared to the control is attributed to the presence of endometriosis.

Further, the invention provides a method of characterizing a gynaecologic condition as being endometriosis, said method providing the following steps:
a) taking a sample from a patient comprising monocytes and/or macrophages, preferably a blood sample,
b) determining and/or quantifying the expression of GPR84 in monocytes and/or macrophages comprised in that sample
c) comparing the obtained parameter with the respective (control) parameter (i) obtained from monocytes and/or macrophages of a healthy patient, or (ii) taken from suitable literature or databases,
wherein increased expression of GPR84 in monocytes and/or macrophages as compared to the control is attributed to the presence of endometriosis.

Further, the invention provides a method of characterizing a gynaecologic condition as being endometriosis, said method providing the following steps:
a) taking a sample from a patient comprising monocytes and/or macrophages, preferably a blood sample,
b) determining and/or quantifying the fraction of GPR84 positive cells in monocytes and/or macrophages comprised in that sample,
c) comparing the obtained parameter with the respective (control) parameter (i) obtained from comprised in that sample of a healthy patient, or (ii) taken from suitable literature or databases.

In all of the above three cases, if the obtained parameter deviates from the control, the gynaecologic condition qualifies as being endometriosis. Preferably, the gynaecologic conditions qualifies as being endometriosis in the case of increased levels as compared to the control parameters determined for MCFFA; or increased expression as compared to the control parameter of GPR84 in monocytes and/or macrophages; or an increased fraction as compared to the control parameter of GPR84 positive cells in monocytes and/or macrophages comprised in that sample are determined. In another embodiment, the determined presence of MCFAA is indicative of the gynaecologic conditions. In such case the parameters qualify or are indicative of the gynaecologic , the inhibitor or antagonist of the present invention may be administered to the patient.

Preferably a MCFFA concentration of more than 3.4 µM is attributed to endometriosis or lessions, more preferably more than 4.0 µM, even more preferred more than 4.5 µM, yet more preferred more than 5.0 µM.

According to one other aspect of the invention, the use of the antagonist or inhibitor disclosed above (for the manufacture of a medicament) in the treatment of a human or animal subject being diagnosed for, suffering from or being at risk of developing endometriosis, or for the prevention of such condition, is provided. Furthermore, a pharmaceutical composition comprising an antagonist or inhibitor according to the above disclosure is provided. Furthermore, a combination of a pharmaceutical composition according to the above disclosure, and one or more other therapeutically active compounds is provided.

Furthermore, a method for treating or preventing endometriosis associated with the undesired expression of GPR84 is provided, which method comprises administering to a subject in need thereof an effective amount of the pharmaceutical composition or the combination according to the above disclosure is provided.

According to one other embodiment of the invention, the antagonist or inhibitor demonstrates pain reducing properties in an *in-vivo* inflamed paws model after administration of complete Freunds' adjuvant.

According to one other aspect of the invention, a method for identifying a compound for use in the treatment and/or prevention of a patient suffering from, at risk of developing, and/or being diagnosed for endometriosis, is provided, which method comprises the screening of one or more test compounds in a GPR84 cAMP release assay on whether they inhibit
- embelin-induced GPR84 activation with an IC₅₀ value of 5 mM or less, preferably 10 µM or less, more preferably 9.4 nM or less; and/or
- 2-OH decanoic-acid-induced GPR84-activation with an IC₅₀ value of 5 mM or less, preferably 10 µM or less, more preferably of 16 nM or less; and/or
- 6-n-octylaminouracil-induced GPR84 activation with an IC₅₀ value of 5 mM or less, preferably 10 µM or less, more preferably 20 nM or less.

In one embodiment a compound is considered as suited for use in said treatment and/or prevention if it inhibits said embelin-induced GPR84 activation with an IC₅₀ value of 5 mM or less, preferably 10 µM or less, more preferably 9.4 nM or less, and/or said 2-OH decanoic-acid-induced GPR84-activation with an IC₅₀ value of 5 mM or less, preferably 10 µM or less, more preferably of 16 nM or less.

According to one other aspect of the invention, a method for identifying a compound for use in the treatment and/or prevention of a patient suffering from, at risk of developing, and/or being diagnosed for endometriosis, is provided, in which method the inhibitory impact of a test compound on
a) a GPR84-ligand or GPR84-agonist stimulated response, or
b) the unstimulated GPR84 activity
is assessed. Preferably, a compound exhibiting an inhibitory impact on said response or activity is considered as suited for said use and/or treatment. According to yet one other aspect of the invention, a method for identifying a compound for use in the treatment and/or prevention of endometriosis in a patient suffering from, at risk of developing, and/or being diagnosed for endometriosis, is provided, wherein the antagonist or inhibitor can be identified by a method comprising the steps of:
a1) contacting a test compound with a GPR84 polypeptide, and
b1) detecting binding of said test compound to said GPR84 polypeptide;
or
a2) determining the activity of a GPR84 polypeptide at a certain concentration of a test compound or in the absence of said test compound, and
b2) determining the activity of said polypeptide at a different concentration of said test compound;
or
a3) determining the activity of a GPR84 polypeptide at a certain concentration of a test compound, and
b3) determining the activity of a GPR84 polypeptide at the presence of a compound known to be a regulator of a GPR84 polypeptide.

These and other embodiments are particularly useful for screening therapeutic compounds by using GPR84 or binding fragments thereof in any of a variety of drug screening techniques. As GPR84 is a G protein coupled receptor any of the methods commonly used in the art may potentially be used to identify GPR84 ligands. For example, the activity of a G protein coupled receptor such as GPR84 can be measured using any of a variety of appropriate functional assays in which activation of the receptor results in an observable change in the level of some second messenger system, such as adenylate cyclase, guanylylcyclase, calcium mobilization, or inositol phospholipid hydrolysis.

Alternatively, the polypeptide or fragment employed in such a test is either free in solution, affixed to a solid support, borne on a cell surface or located intracellular. One method of drug screening utilizes eukaryotic or prokaryotic host cells which are stably transformed with recombinant nucleic acids expressing the polypeptide or fragment. Drugs are screened against such transformed cells in competitive binding assays. Such cells, either in viable or fixed form, are used for standard binding assays.

Measured, for example, is the formation of complexes between GPR84 and the agent. Alternatively, one may examine the diminution in complex formation between GPR84 and a ligand caused by the agent being tested.

Thus, the present invention provides methods of screening for drug candidates, drugs, or any other agents which affect GPR84 signal transduction. These methods, well known in the art, comprise contacting such an agent with the GPR84 polypeptide or a fragment thereof and assaying (i) for the presence of a complex between the agent and GPR84 polypeptide or fragment, or (ii) for the presence of a complex between GPR84 polypeptide or fragment and the cell. In such competitive binding assays, the GPR84 polypeptide or fragment is typically labeled. After suitable incubation, free GPR84 polypeptide or fragment is separated from that present in bound form, and the amount of free or uncomplexed label is a measure of the ability of the particular agent to bind to GPR84 or to interfere with the GPR84-agent complex.

Another technique for drug screening provides high throughput screening for compounds having suitable binding affinity to GPR84 polypeptides. Briefly stated, large numbers of different small peptide test compounds are synthesized on a solid substrate, such as plastic pins or some other surface. The peptide test compounds are reacted with GPR84 polypeptide and washed. Bound GPR84 polypeptide is then detected by methods well known in the art. Purified GPR84 are also coated directly onto plates for use in the aforementioned drug screening techniques. In addition, non-neutralizing antibodies are used to capture the peptide and immobilize it on the solid support.

Another technique is the cAMP release as further described herein above and in the Example's experiments as a high-througput screen.

This invention also contemplates the use of competitive drug screening assays in which neutralizing antibodies capable of binding GPR84 specifically compete with a test compound for binding to GPR84 polypeptides or fragments thereof. In this manner, the antibodies are used to detect the presence of any peptide which shares one or more antigenic determinants with GPR84.

According to one other aspect of the invention, a method for identifying a compound for use in the treatment and/or prevention of a patient suffering from, at risk of developing, and/or being diagnosed for endometriosis, is provided, which method comprises the steps of:
a) providing an endometrial slice assay using eutopic or ectopic endometrium,
b) treating slices with a GPR84 agonist in the presence or absence of the test compound, and
c) determining whether the test compound reduces GPR84-agonist-induced cytokine, growth factors and/or chemokine expression and/or secretion.

In a preferred embodiment, reduction a reduced expression and secretion in step c) is attributed to the compound being suited in said use in treatment and/or prevention.

Said cytokines, growth factors, and chemokines include, but are not limited to, IL-1beta as well as TNFalpha. Examples of this approach are shown in the experimental section. Said agonists include medium-chain fatty acids, preferably fully saturated medium-chain fatty acids, more preferably decanoic acid.

According to one other aspect of the invention, a method for identifying a compound for use in the treatment and/or prevention of endometriosis in a patient suffering from, at risk of developing, and/or being diagnosed for endometriosis, is provided, which method comprising the steps of:
a) providing a preparation of human peritoneal mononuclear cells or GPR84 expression monocytes cell line, such as THP1 or U937
b) treating cells with the test compound, and
c) determining whether the test compound reduces release of chemokines inducing chemotaxis of peripheral blood mononuclear cells, preferably IL-1 beta and TNFalpha.

In a preferred embodiment reduction of said release of chemokines inducing chemotaxis, preferably IL-1beta and TNFalpha, is attributed to the compound being suited in said use in treatment and/or prevention.

GPR84 expression monocytes cell line, such as THP1 or U937, are known by those skilled in the art [see 4, and 9, which are incorpoarated herein by reference].

Preferably, in step b) the inhibition of release or expression of a messenger molecule indicative of the activity of GPR84 polypeptide is measured. Preferably, said method is one of the methods disclosed in US2015330968A1, the content of which is incorporated by reference herein. Preferably, said test compound is selected from the group consisting of capric acid, undecanoic acid, lauric acid, 2,5-Dihydroxy-3-undecyl-2,5-cyclohexadiene-1,4-dione (Embelin), icosa-5,8,11,14-tetraynoic acid, 5S,6R-Dihydroxy-icosa-7,9,11,14-tetraynoic acid, diindorylmethane and indol-3-carbinol. Preferably, said messenger molecule is cyclic AMP (cAMP).

### EXAMPLES

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. Any reference signs should not be construed as limiting the scope.

All amino acid sequences disclosed herein are shown from N-terminus to C-terminus; all nucleic acid sequences disclosed herein are shown 5'->3'.

### Experiments

### In vitro assay 1: GPR84 ligand and antagonist characterization

Dose response experiments for GPR84 agonists were carried out using the cAMP Hunter^{™} CHO-K1 GPR84 Gi cell system (DiscoverX) (PathHunter® CHO-K1 hGPR84 β-Arrestin Cell Line cultured with DMF12 HAM, 9% FBS; 300ug/ml Hygromycin; 800ug/ml Geneticin; 2mM Glut; 1% Pen / Strep) in a 384-well plate format. Cell viability and number were determined (Omni Life Science; Casy Cell Counter/Analyzer) prior to seeding and agonist treatment was followed by a 60-minute incubation time to determine cAMP levels using time-resolved FRET (TR-FRET) on a high-throughput BMG Labtech Pherastar instrument. For antagonist dose-responses cells were incubated in the presence of antagonists for 30 minutes prior to adding agonists as described in table 1. Following a 60-minute incubation cAMP levels were determined by TR-FRET as described above. Data were fitted using GraphPad Prism.

The data obtained are summarized in Table 1:

**Table 1:**

| **Example** | **Agonism EC₅₀ [M]** | **Antagonism IC₅₀ [M]** |
|---|---|---|
| Embelin | 3.8 x 10⁻⁶ M | n.a. |
| Decanoic acid | 10 x 10⁻⁶ M | n.a. |
| 2-OH- decanoic acid | 24 x 10⁻⁶ M | n.a. |
| CMPD 139 | n.a. | 20 x 10⁻⁹M |
| (with 6-n-octylaminouracil 1.34 X 10⁻⁷M) | | |
| CMPD 139 | n.a. | 9.4 x 10⁻⁹M |
| (with embelin 6.00 10⁻⁶M) | | |
| CMPD 139 | n.a. | 16 x 10⁻⁹M |
| (with 40 x 10⁻⁶ M 2-OH decanoic acid | | |

| | | |
|---|---|---|
| n.a. : not applied | | |

### Ex vivo Assay 1: GPR84 ligand associated endometrial inflammation assay

Human eutopic endometrium tissue biopsies as surrogate for endometriosis lesion were cut into small slices, washed with HBSS buffer [ThermoFischer, 14175095] and incubated in DMEM/F12 medium [ThermoFischer, 11320-033], supplemented with 5% FCS for 3 hours. Medium was removed and replaced with DMEM/F12, 2% CCS (charcoal stripped) supplemented with combinations of GPR84 agonist (Decanoic Acid) and the GPR84 antagonist CMPD139. After 21 hours cytokines in the supernatant were quantified with the Bio-Plex platform according to the supplier's instructions. Data were analyzed using the GraphPad Software (La Jolla, USA).
In this setting, GPR84 ligands surprisingly increased the levels of several pro inflammatory cytokines and growth factors previously established to be relevant in endometriosis. These mediators included IL1β and TNFalpha. Treatment with the antagonist CMPD 139 at 10⁻⁵ M combined with decanoic acid as an agonist reduced all measured cytokine and growth factors as compared to the treatment with an agonist of GPR84 alone. Results are shown in Table 2. Thus, activation of GPR84 contributes to the inflammation associated with endometriosis and antagonists are able to reduce inflammation

**Table 2:**

| **Sample** | **Vehicle** | | **Decanoic Acid (10 µM)** | | **Decanoic Acid (10 µM) + CMPD. 139 (10 µM)** | |
|---|---|---|---|---|---|---|
| | **pg**/**mL** | **+/-SD** | **pg**/**mL** | **+/- SD** | **pg/mL** | **+/- SD** |
| **IL-1beta** | 0,7467 | 0,5829 | 1,413 | 0,6352 | 0,9600 | 0,7923 |
| **TNFalpha** | 1,000 | 0,7835 | 1,953 | 0,6087 | 1,270 | 0,8648 |

### In vitro Assay 2: GPR84 ligand associated ILbeta and TNFalpha release from PBMCs

PBMCs were isolated by Ficoll-Paque Plus (Biochrom) density gradient centrifugation from whole blood. Briefly, PBMCs were centrifuged at 250g for 30 sec and resuspended and cultured in RPMI 1640 medium [Gibco, CAT No. 11875093] with 10% heat-inactivated FBS, L-Glutamine (10 g/L), penicillin (100 IU/mL), and streptomycin (100 ng/mL) at 37 °C in a 5% CO₂ environment. Cells were stimulated with LPS (100 ng/ml, Sigma-Aldrich) for 24 hours and treated with the GPR84 agonist 6-n-octylaminouracil and antagonist CMPD 139 for 4h. IL-1beta and TNFalpha concentrations were analyzed by ELISA (Mesoscale Discovery, Germany). CMPD139 reduced GPR84 elevated IL-1beta and TNFalpha levels as shown in Table 3

**Table 3:**

| **Sample** | **Vehicle** | | **6-n-octylaminouracil (10 µM)** | | **6-n-octylaminouracil (10 µM)+ CMPD 139 (10 µM)** | |
|---|---|---|---|---|---|---|
| | **pg/ml** | **+/- SD** | **pg/ml** | **+/- SD** | **pg/ml** | **+/- SD** |
| **IL-1beta** | 2472,868 | 249,516 | 3427,673 | 1148,718 | 1933,011 | 326,9045 |
| **TNF alpha** | 641,0958 | 245,5852 | 955,6923 | 164,9221 | 479,6307 | 26,15715 |

### Immunohistochemistry of peritoneal tissue

Cross sections of formalin (3.7%)-fixed, paraffin-embedded tissue was deparaffinized, hydrated and H&E [Haematoxylin and Eosin stain] stained using an automatic Leica Multistainer and Coverslipper (Leica ST5020+CV 5030, Leica Microsystems, Germany).

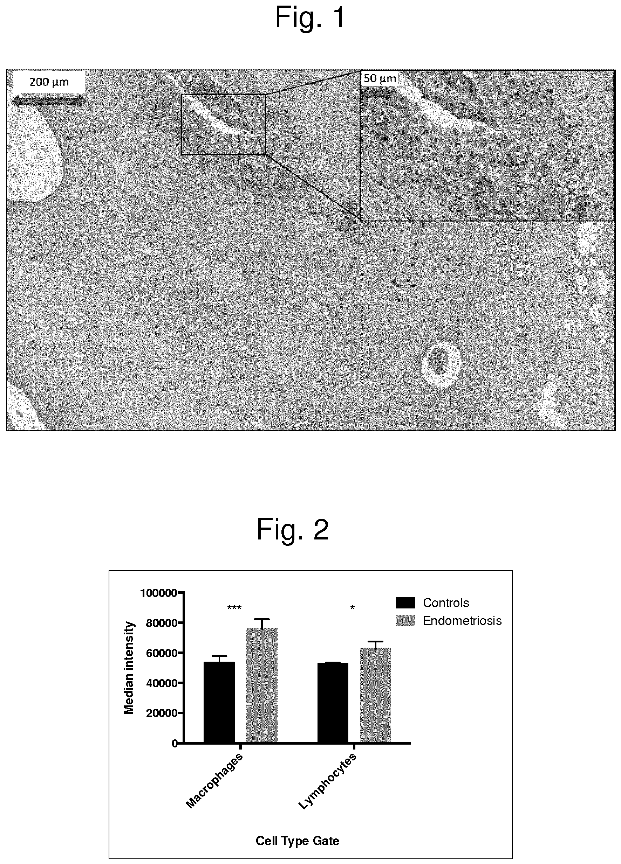

For detection of GPR84- and CD68-expressing cells, anti-GPR84-IgG (A91742, rabbit polyclonal, Sigma-Aldrich, Germany) and anti-CD68-IgG (KP1, mouse monoclonal, Abcam, Germany) were used as primary antibodies, respectively. The Dako Envision+ System-HRP (Dako, USA) and GBI permanent red (GBI, USA) substrate solution were applied to develop specific staining. Slides were additionally counter-stained with H&E. Fig. 1 is a photomicrograph of such slide.

Immunohistochemistry of GPR84 expression in ectopic lesions of endometriosis. Ectopic peritoneal lesions are rich in infiltrating myeloid cells (CD68) and co-stain with GPR84.

### Determination of GPR84 positive cells in the peritoneal cavity of endometriosis patients vs. controls

Mononuclear cells from peritoneal fluid (n = 3 per group) were collected by ficoll density gradient centrifugation and frozen at -80°C. To measure GPR84 expression, cells were thawed on ice for 30 min and washed with PBS. Cells were then stained with Fixable Viability Dye eFluor® 780 (eBioscience, cat. #65-0865) according to manufacturer's instructions, followed by fixation with 1% Fixation buffer (BioLegend, cat. # 420801). Fc receptors were blocked by 20% human serum and 5µL/100µL Human TruStain FcX Fc Receptor Blocking serum (BioLegend, cat. # 422301), and cells were permeabilised with PBS supplemented with 1% BSA and 0.1% saponin. Permeabilised cells were then incubated with GPR84 antibody (Bioss, cat. # bs-15353R) and rabbit IgG isotype control (Bioss, cat no# bs-0295P-PE) for 1 hour on ice. After staining, cells were washed twice and acquired on a flow cytometer (BD LSRFORTESSA; BD Bioscience).

Flow cytometry analysis showed higher expression levels of GPR84 in macrophages from endometriosis patients vs. control patients (Fig. 2).

### Determination of MCFA in endometrial tissue

The content of individual free medium-chain fatty acids (MCFA; decanoic acid and myristic acid) in eutopic (endometrium) and ectopic (lesion) endometrial tissue was determined as their corresponding methyl ester derivatives using gas chromatography coupled with mass spectrometric (GC/MS) detection (Agilent 7890 GC/5975 MSD). Samples were treated with methanolic HCl solution for a prolonged time period to completely convert free fatty acids into their methyl esters. Chromatograms in Selected Ion Monitoring (SIM) mode with four characteristic ions were recorded for quantitation of individual methylester derivatives. Decanoic acid and myristic acid level are higher in endometrial lesion (Lesions) compared to eutopic endometrium (Controls). Statistically analysis; unpaired t-test, significantly difference (P < 0.05).

**Table 4: Concentrations of MCFA**

| **Sample** | **Decanoic Acid** | | **Myristic Acid** | |
|---|---|---|---|---|
| | **µM** | **+/- SD** | **µM** | **+/- SD** |
| Lesions (n = 5) | 0,5103 | 0,1902 | 6,596 | 0,3424 |
| Controls (n=5) | 0,3185 | 0,2019 | 6,784 | 0,6641 |

### Effects of CMPD139 in the CFA pain model

The efficacy of CMPD139 *in vivo* on inflammatory pain was measured in inflamed paws after administration of complete Freunds' adjuvans (CFA) (24h) in the dynamic weight-bearing (DWB) model [10]. The effects of repeated preventive treatment with CMPD139 on pain following repeated oral and subcutaneous administration (3x) in the mouse CFA model of inflammation were investigated using a preventive setting. The GPR84 antagonist CMPD139 (90 or 30 mg/kg, p.o.+s.c., 3x doses) was administered 2h before injection of CFA and 6-8h later at day 0. At 24h after CFA application, the third dose of CMPD139 was given 2h before DWB testing. Statistical analysis was performed with one-way analysis of variance, followed by Bonferroni's multiple comparison test against vehicle control groups using the GraphPad PRISM software, *p<0.05.

**Table 5:**

| **Example** | **mg/kg p.o./s.c.** | **Weight distribution(% Ipsi/contra)** |
|---|---|---|
| | | **2h post last dose mean +/- SD** |
| Vehicle | | 42.91 +/- 19.32 |
| CMPD139 | 3x 90+90mg | 60.37 +/- 15.45* |
| CMPD139 | 3x 30+ 30mg | 55.82 +/-17.38 |

### References

The following articles are referred to in this specification. For enablement purposes of the present invention, the content thereof is incorporated herein by reference.
1. M. Hickey, K. Ballard, C. Farquhar, Endometriosis. BMJ 348, g1752 (2014).
2. J. A. Sampson, Peritoneal endometriosis due to the menstrual dissemination of endometrial tissue into the peritoneal cavity. Am J Obstet Gynecol 14, 422-469 (1927).
3. C. H. Syrop, J. Halme, Peritoneal fluid environment and infertility. Fertil Steril 48, 1-9 (1987).
4. S. H. Ahn, S. P. Monsanto, C. Miller, S. S. Singh, R. Thomas, C. Tayade, Pathophysiology and Immune Dysfunction in Endometriosis. Biomed Res Int 2015, 795976 (2015).
5. D. P. Braun, H. Gebel, R. House, N. Rana, N. P. Dmowski, Spontaneous and induced synthesis of cytokines by peripheral blood monocytes in patients with endometriosis. Fertil Steril 65, 1125-1129 (1996).
6. H. N. Ho, M. Y. Wu, Y. S. Yang, Peritoneal cellular immunity and endometriosis. Am J Reprod Immunol 38, 400-412 (1997).
7. J. Wieseler-Frank, S. F. Maier, L. R. Watkins, Central proinflammatory cytokines and pain enhancement. Neurosignals 14, 166-174 (2005).
8. M. Morotti, K. Vincent, J. Brawn, K. T. Zondervan, C. M. Becker, Peripheral changes in endometriosis-associated pain. Hum Reprod Update 20, 717-736 (2014).
9. E. Alvarez-Curto, G. Milligan, Metabolism meets immunity: The role of free fatty acid receptors in the immune system. Biochem Pharmacol, (2016).
10. J. E. Lattin, K. Schroder, A. I. Su, J. R. Walker, J. Zhang, T. Wiltshire, K. Saijo, C. K. Glass, D. A. Hume, S. Kellie, M. J. Sweet, Expression analysis of G Protein-Coupled Receptors in mouse macrophages. Immunome Res 4, 5 (2008).
11. J. Xu, H. Morinaga, D. Oh, P. Li, A. Chen, S. Talukdar, Y. Mamane, J. A. Mancini, A. R. Nawrocki, E. Lazarowski, J. M. Olefsky, J. J. Kim, GPR105 ablation prevents inflammation and improves insulin sensitivity in mice with diet-induced obesity. J Immunol 189, 1992-1999 (2012).
12. S. Talukdar, J. M. Olefsky, O. Osborn, Targeting GPR120 and other fatty acid-sensing GPCRs ameliorates insulin resistance and inflammatory diseases. Trends Pharmacol Sci 32, 543-550 (2011).
13. A. Steinert, K. Radulovic, J. Niess, Gastro-intestinal tract: The leading role of mucosal immunity. Swiss Med Wkly 146, w14293 (2016).
14. K. M. Maslowski, A. T. Vieira, A. Ng, J. Kranich, F. Sierro, D. Yu, H. C. Schilter, M. S. Rolph, F. Mackay, D. Artis, R. J. Xavier, M. M. Teixeira, C. R. Mackay, Regulation of inflammatory responses by gut microbiota and chemoattractant receptor GPR43. Nature 461, 1282-1286 (2009).
15. N. Singh, A. Gurav, S. Sivaprakasam, E. Brady, R. Padia, H. Shi, M. Thangaraju, P. D. Prasad, S. Manicassamy, D. H. Munn, J. R. Lee, S. Offermanns, V. Ganapathy, Activation of Gpr109a, receptor for niacin and the commensal metabolite butyrate, suppresses colonic inflammation and carcinogenesis. Immunity 40, 128-139 (2014).
16. D. Y. Oh, S. Talukdar, E. J. Bae, T. Imamura, H. Morinaga, W. Fan, P. Li, W. J. Lu, S. M. Watkins, J. M. Olefsky, GPR120 is an omega-3 fatty acid receptor mediating potent anti-inflammatory and insulin-sensitizing effects. Cell 142, 687-698 (2010).
17. M. Suzuki, S. Takaishi, M. Nagasaki, Y. Onozawa, I. lino, H. Maeda, T. Komai, T. Oda, Medium-chain fatty acid-sensing receptor, GPR84, is a proinflammatory receptor. J Biol Chem 288, 10684-10691 (2013).
18. C. Bouchard, J. Page, A. Bedard, P. Tremblay, L. Vallieres, G protein-coupled receptor 84, a microglia-associated protein expressed in neuroinflammatory conditions. Glia 55, 790-800 (2007).
19. J. Wang, X. Wu, N. Simonavicius, H. Tian, L. Ling, Medium-chain fatty acids as ligands for orphan G protein-coupled receptor GPR84. J Biol Chem 281, 34457-34464 (2006).
20. C. Venkataraman, F. Kuo, The G-protein coupled receptor, GPR84 regulates IL-4 production by T lymphocytes in response to CD3 crosslinking. Immunol Lett 101, 144-153 (2005).
21. L. S. Nicol, J. M. Dawes, F. La Russa, A. Didangelos, A. K. Clark, C. Gentry, J. Grist, J. B. Davies, M. Malcangio, S. B. McMahon, The role of G-protein receptor 84 in experimental neuropathic pain. J Neurosci 35, 8959-8969 (2015).
22. M. C. Hunt, M. I. Siponen, S. E. Alexson, The emerging role of acyl-CoA thioesterases and acyltransferases in regulating peroxisomal lipid metabolism. Biochim Biophys Acta 1822, 1397-1410 (2012).
23. A. Laux-Biehlmann, J. Boyken, H. Dahllof, N. Schmidt, T. M. Zollner, J. Nagel, Dynamic weight bearing as a non-reflexive method for the measurement of abdominal pain in mice. Eur J Pain 20, 742-752 (2016).
24. A. Laux-Biehlmann, T. d'Hooghe, T. M. Zollner, Menstruation pulls the trigger for inflammation and pain in endometriosis. Trends Pharmacol Sci 36, 270-276 (2015).
25. C. Southern et al., Screening β-arrestin recruitment for the identification of natural ligands for orphan G-protein-coupled receptors. J Biomol Screen, 18 (5): 599-609 (2013)
26. B. X. Chen, B.F. Erlanger, Intracellular delivery of monoclonal antibodies. Immunol Lett 5;88(2): 87 (2003)
27. G.Y. Berguig et al., Intracellular delivery system for antibody-Peptide drug conjugates. Mol Ther May;23(5):907-17 (2015)
28. L. Meunier. Optimizing the Potential of Intracellular Therapeutic Antibodies - Executive Interview - BioCellChallenge: Drug Dev. April (2014)
29. C. Xu, J. Wang, Delivery systems for siRNA drug development in cancer therapy. Asian Journal of Pharmaceutical Sciences 10 (1), 1-12 (2015)
30. G. Köhler, C. Milstein, Continuous cultures of fused cells secreting antibody of predefined specificity, Nature 256, 495-497 (1975)
31. M. Jinek et al., A programmable dual-RNA-guided DNA endonuclease in adaptive bacterial Immunity. Science 17; 337(6096): 816-21 (2012),
32. H. Yin et al., Therapeutic genome editing by combined viral and non-viral delivery of CRISPR system components in vivo. Nature Biotechnology 34,328-333. (2016)
33. B. Zetsche et al., Cpf1 is a single RNA-guided endonuclease of a class 2 CRISPR-Cas system. Cell 22;163(3):759-71 (2015)
34. M. Blind, M. Blank. Aptamer Selection Technology and Recent Advances. Molecular Therapy Nucleic Acids 4, e223 (2015)
35. K. W. Thiel, P. H. Giangrande. Intracellular delivery of RNA-based therapeutics using aptamers. Ther Deliv. 1(6):849-61 (2010)

### SEQ ID NO 1 - G-protein coupled receptor 84

MWNSSDANFSCYHESVLGYRYVAVSWGVVVAVTGTVGNVLTLLALAIQPKLRTRFN
LLIANLTLADLLYCTLLQPFSVDTYLHLHWRTGATFCRVFGLLLFASNSVSILTLCLIAL
GRYLLIAHPKLFPQVFSAKGIVLALVSTWVVGVASFAPLWPIYILVPVVCTCSFDRIRG
RPYTTILMGIYFVLGLSSVGIFYCLIHRQVKRAAQALDQYKLRQASIHSNHVARTDEA
MPGRFQELDSRLASGGPSEGISSEPVSAATTQTLEGDSSEVGDQINSKRAKQMAE
KSPPEASAKAQPIKGARRAPDSSSEFGKVTRMCFAVFLCFALSYIPFLLLNILDARVQ
APRVVHMLAANLTWLNGCINPVLYAAMNRQFRQAYGSILKRGPRSFHRLH

## Claims

1. An antagonist or inhibitor of G protein-coupled receptor 84 (GPR84) for use in the treatment and/or prevention of endometriosis in a patient
• suffering from,
• at risk of developing, and/or
• being diagnosed for
endometriosis.

2. The antagonist or inhibitor according to claim 1, which is an antibody, preferably a monoclonal antibody, specifically binding to GPR84, or is an antigen-binding fragment or derivative thereof.

3. The antagonist or inhibitor according to claim 1, which is a nucleic acid inhibitor.

4. The antagonist or inhibitor according to claim 3, which comprises a nucleic acid molecule that specifically binds to at least a substretch of a polynucleotide which encodes a GPR84 protein.

5. The antagonist or inhibitor according to claim 3 or 4, wherein the polynucleotide encodes for a GPR84 protein which comprises an AA sequence as disclosed in Uniprot under reference Number Q9NQS5, preferably according to SEQ ID NO: 1.

6. The antagonist or inhibitor according to claim 3, which is an aptamer.

7. The antagonist or inhibitor according to claim 1, which is a small molecule.

8. The antagonist or inhibitor according to any one of the aforementioned claims, wherein the GPR84 protein which is inhibited or antagonized by the antibody, aptamer or small molecule comprises an AA sequence as disclosed in Uniprot under reference Number Q9NQS5, preferably according to SEQ ID NO: 1.

9. The antagonist or inhibitor according to any one of the aforementioned claims, which inhibits
• embelin-induced GPR84 activation with an IC₅₀ value of 5 mM or less, preferably 10 µM or less, more preferably 9.4 nM or less, and/or
• 2-OH decanoic-acid-induced GPR84-activation with an IC₅₀ value of 5 mM or less, preferably 10 µM or less, more preferably of 16 nM or less, and/or
• 6-n-octylaminouracil-induced GPR84 activation with an IC₅₀ value of 5 mM or less, preferably 10 µM or less, more preferably 20 nM or less.
as determined in a GPR84 cAMP release assay.

10. The antagonist or inhibitor according to any one of claims 1, and 7 to 9, which is a member of one of the groups selected from the list comprising:
• Dihydropyridoisoquinolinone
• Dihydropyrimidinoisoquinolinone

11. The antagonist of any one of the aforementioned claims, wherein the endometriosis is **characterized by**
• presence of medium chain free fatty (MCFFA) acids, and/or acyl-CoA thioesterase enyzmes (ACOT) and/or fatty acid synthases (FASN) in ectopic lesions, as compared to eutopic endometrium; and/or
• increased expression of GPR84 in macrophages taken from an endometriosis patient, as compared to macrophages taken from a control patient; and/or
• larger fraction of GPR84 positive cells in monocytes and/or macrophages taken from an endometriosis patient, as compared to monocytes and/or macrophages taken from a control patient.

12. The antagonist for use of any of the aforementioned claims, wherein said treatment is the treatment of endometrioses associated inflammatory pain.

13. Use of the antagonist or inhibitor according to any of the aforementioned claims (for the manufacture of a medicament) in the treatment of a human or animal subject being diagnosed for, suffering from or being at risk of developing endometriosis, or for the prevention of such condition.

14. A pharmaceutical composition comprising an antagonist or inhibitor according to any one of claims 1 to 12.

15. A combination of a pharmaceutical composition according to claim 14, and one or more other therapeutically active compounds.

16. A method for treating or preventing endometriosis associated with the undesired expression of GPR84, comprising administering to a subject in need thereof an effective amount of the pharmaceutical composition according to claim 14 or the combination according to claim 15.

17. A method for identifying a compound for use in the treatment and/or prevention of endometriosis in a patient suffering from, at risk of developing, and/or being diagnosed for endometriosis, which method comprises the screening of one or more test compounds in a GPR84 cAMP release assay on whether they inhibit
• embelin-induced GPR84 activation with an IC₅₀ value of 5 mM or less, preferably 10 µM or less, more preferably 9.4 nM or less, and/or
• 2-OH decanoic-acid-induced GPR84-activation with an IC₅₀ value of 5 mM or less, preferably 10 µM or less, more preferably of 16 nM or less, and/or
• 6-n-octylaminouracil-induced GPR84 activation with an IC₅₀ value of 5 mM or less, preferably 10 µM or less, more preferably 20 nM or less.

18. The method according to claim 17, wherein a compound is considered as suited for use in said treatment and/or prevention if it inhibits said embelin-induced GPR84 activation with an IC₅₀ value of 5 mM or less, most preferably 9.4 nM or less, and/or said 2-OH decanoic-acid-induced GPR84-activation with an IC₅₀ value of 5 mM or less, most preferably of 16 nM or less.


SEQUENCE LISTING

<110>  Bayer Pharma AG

<120>  Inhibitors and antagonists of GPR84 for the treatment of
       endometriosis

<130>  163084

<160>  1

<170>  PatentIn version 3.5

<210>  1
<211>  396
<212>  PRT
<213>  G-Protein coupled receptor 84

<400>  1

Met Trp Asn Ser Ser Asp Ala Asn Phe Ser Cys Tyr His Glu Ser Val
1               5                   10                  15

Leu Gly Tyr Arg Tyr Val Ala Val Ser Trp Gly Val Val Val Ala Val
            20                  25                  30

Thr Gly Thr Val Gly Asn Val Leu Thr Leu Leu Ala Leu Ala Ile Gln
        35                  40                  45

Pro Lys Leu Arg Thr Arg Phe Asn Leu Leu Ile Ala Asn Leu Thr Leu
    50                  55                  60

Ala Asp Leu Leu Tyr Cys Thr Leu Leu Gln Pro Phe Ser Val Asp Thr
65                  70                  75                  80

Tyr Leu His Leu His Trp Arg Thr Gly Ala Thr Phe Cys Arg Val Phe
                85                  90                  95

Gly Leu Leu Leu Phe Ala Ser Asn Ser Val Ser Ile Leu Thr Leu Cys
            100                 105                 110

Leu Ile Ala Leu Gly Arg Tyr Leu Leu Ile Ala His Pro Lys Leu Phe
        115                 120                 125

Pro Gln Val Phe Ser Ala Lys Gly Ile Val Leu Ala Leu Val Ser Thr
    130                 135                 140

Trp Val Val Gly Val Ala Ser Phe Ala Pro Leu Trp Pro Ile Tyr Ile
145                 150                 155                 160

Leu Val Pro Val Val Cys Thr Cys Ser Phe Asp Arg Ile Arg Gly Arg
                165                 170                 175

Pro Tyr Thr Thr Ile Leu Met Gly Ile Tyr Phe Val Leu Gly Leu Ser
            180                 185                 190

Ser Val Gly Ile Phe Tyr Cys Leu Ile His Arg Gln Val Lys Arg Ala
        195                 200                 205

Ala Gln Ala Leu Asp Gln Tyr Lys Leu Arg Gln Ala Ser Ile His Ser
    210                 215                 220

Asn His Val Ala Arg Thr Asp Glu Ala Met Pro Gly Arg Phe Gln Glu
225                 230                 235                 240

Leu Asp Ser Arg Leu Ala Ser Gly Gly Pro Ser Glu Gly Ile Ser Ser
                245                 250                 255

Glu Pro Val Ser Ala Ala Thr Thr Gln Thr Leu Glu Gly Asp Ser Ser
            260                 265                 270

Glu Val Gly Asp Gln Ile Asn Ser Lys Arg Ala Lys Gln Met Ala Glu
        275                 280                 285

Lys Ser Pro Pro Glu Ala Ser Ala Lys Ala Gln Pro Ile Lys Gly Ala
    290                 295                 300

Arg Arg Ala Pro Asp Ser Ser Ser Glu Phe Gly Lys Val Thr Arg Met
305                 310                 315                 320

Cys Phe Ala Val Phe Leu Cys Phe Ala Leu Ser Tyr Ile Pro Phe Leu
                325                 330                 335

Leu Leu Asn Ile Leu Asp Ala Arg Val Gln Ala Pro Arg Val Val His
            340                 345                 350

Met Leu Ala Ala Asn Leu Thr Trp Leu Asn Gly Cys Ile Asn Pro Val
        355                 360                 365

Leu Tyr Ala Ala Met Asn Arg Gln Phe Arg Gln Ala Tyr Gly Ser Ile
    370                 375                 380

Leu Lys Arg Gly Pro Arg Ser Phe His Arg Leu His
385                 390                 395